# EUROPEAN PATENT APPLICATION

(11) **EP 4 166 105 A1**
(43) Date of publication of application: **19.04.2023**
(21) Application number: 21829949.3
(22) Date of filing: 25.06.2021
(51) Int. Cl.: A61B 18/02, A61B 18/12, A61B 18/14, A61B 10/02

(54) **ABLATION PROBE HAVING SEPARABLE OUTER SLEEVE AND FREEZING FUNCTION, AND METHOD**

(30) Priority: 26.06.2020 CN 202010593103
(71) Applicant: Tianjin Meidian Medical Technology Co., Ltd., Tianjin 300384 (CN)
(72) Inventor: ZHAO, Guojiang, Tianjin 300384 (CN); YUE, Honglei, Tianjin 300384 (CN); SONG, Zihao, Tianjin 300384 (CN)
(74) Representative: Groth & Co. KB
(86) International application number: PCT/CN2021/102524
(87) International publication number: WO 2021/259379

(57) **Abstract**

An ablation probe having a separable outer sleeve and a freezing function, comprising: an inner probe (101, 201, 301, 401), an outer sleeve (102, 202, 302, 402), and a handle (105, 205, 305, 405). The inner probe (101, 201, 301, 401) is slidably connected to the outer sleeve (102, 202, 302, 402); one end of the inner probe (101, 201, 301, 401) is fixed to one end of the handle (105, 205, 305, 405); said ablation probe further comprises a separating assembly; the separating assembly comprises a first separable member (103) and a second separable member (104); the first separable member (103) and the second separable member (104) are detachably connected; the first separable member (103) is fixed to the outer sleeve (102, 202, 302, 402); the second separable member (104) is fixed to the handle (105, 205, 305, 405); the outer sleeve (102, 202, 302, 402) is a heat-insulating tube. The slide of the outer sleeve (102, 202, 302, 402) on the surface of the inner probe (101, 201, 301, 401) can change the size and distribution of the ablation area of the inner probe (101, 201, 301, 401); the separable structure can form a channel between the outside of the body and the target area of treatment by means of the outer sleeve (102, 202, 302, 402) for biopsy, drug delivery, and hemostasis; the separable structure can be designed into a non-reusable structure for one-time use only to improve the safe usage of the ablation probe..

## Description

### CROSS-REFERRED APPLICATIONS

This application requires the priority of a Chinese patent application filed on June 26, 2020 with application number 2020105931039 and the title of the invention "Ablation probe with a split outer sleeve and freezing function and method of use of the same ", the entire content of which is combined by reference in this application.

### TECHNICAL FIELD

The invention relates to an ablation probe, in particular an ablation probe having a separable outer sleeve and freezing function and a method thereof.

### BACKGROUND

Tissue and tumor ablation techniques are the emerging physiotherapy methods in recent years, with the advantages of safe, effective, minimally invasive and definite efficacy, and are widely used in the local ablation therapy of various tissues and tumors. The design and application of ablation probes is one of the cores of this technology, determining its ablation capability, minimally invasive performance, safety, and effectiveness.

The ablation target area requires positioning puncture, and sometimes biopsy and injection of the target area. Generally, device accessories, such as expanding sheath, are needed, but expanding sheath increases the cost of operation, is inconvenient in use and will greatly enlarge the surgical wound, which has many adverse effects on tumor ablation surgery. Ablation probes often require a thermal or electrical insulating structure, which is generally set inside the probe that increases the diameter of the ablation probe, and also hinders the formation of the ice ball, affecting the cryoablation effect.

### SUMMARY

The present invention is to propose an ablation probe with a split outer sleeve to solve the inability to regulate the size and distribution of the inner probe ablation area, and the problem that requires the use of various devices to complete the expansion, medication, puncture biopsy and ablation of the target area.

To solve the above technical problems, the technical scheme of the present invention is an ablation probe with a split outer sleeve and freezing function, including an inner probe, an outer sleeve and a handle. The inner probe is slipped connected to the outer sleeve, and one end of the inner probe is fixed with one end of the handle. Further comprising a split assembly, the split assembly comprising a first split member and a second split member. The first split member and the second split member are detachable, the first split member is fixed with the outer sleeve, the second split member is partially fixed with the handle; and the outer sleeve is an insulation tube; The inner probe is either a cryoablation probe or a cryoelectric ablation probe with no thermal insulation structure.

Further, inner probe is either a cryoablation probe or a cryoelectric ablation probe.

Further, the thermal insulation tube is provided with an electrical insulating layer.

Further, the distal end of the outer sleeve is provided with at least one electrode, with portions other than the electrode with an insulating structure.

Further, the surface of the inner probe is provided with marked scale.

Further, the ablation probe further comprises an outer sheath, one end of the outer sheath fixed to the first split member, the outer sheath further comprising a sliding knob fixed through the groove to the outer sleeve.

Further, the outer sheath is provided with a scale and a limit slot.

Further, the detachable structure of the first split member and the second split member is a reusable structure.

Further, the reusable structure is a stuck structure, a spin structure, or a surplus mating structure.

Further, the detachable connection structure of the first split member and the second split member is a non-reusable structure.

Further, the non-reusable structure is a torsional, crushing, or pull-off structure.

Further, the inner probe, the first and the second split members are flexible structures, and the handle and the inner probe are provided in one.

Further, the first split member is a balloon connector, the distal end of the inner probe is provided with at least one first gas hole and a second gas hole, the first gas hole is used to input the fluid inside the inner probe into the balloon connector, and the second gas hole is used to return the fluid inside the balloon connection into the inner probe.

Further, the balloon is at least partially conductive or the surface is provided with a conductive portion.

Further, the first split member is a thermocouple connector, the thermalcouple connector can produce a thermal effect, and the thermal effect can be used for thawing or thermal ablation.

Further, the thermocouple connector is used for temperature measurement.

Further, the surface of the second split member is provided with at least one electrode.

Further, the surface of the first split member is provided with at least one electrode.

An ablation method of a ablation probe with a split outer sleeve and freezing function, by placing the ablation probe into the target area, separating the first and second split members and extracting the inner probe to form the channel with the target area, injecting the agent into or through the sleeve, connecting the first split member or the second split member to adjust the ablation area, and starting the ablation to ablate the target area.

Further, the first and the second split members are separated and the inner probe is removed and the agent is re-injected into the sleeve.

The advantages and positive effects of the present invention are:
(1) The split structure can form a channel to the therapeutic target in vitro through the outer sleeve, which can deliver drugs with beneficial effects to the target area.
(2) The split structure can be designed as a non-reusable structure, and the safety of the ablation probe can be improved for single-use only.
(3) It can be used with a variety of ablation methods with strong applicability.
(4) The slip of the outer sleeve changes the length of the insulating layer covering the inner probe, thus changing the size and length of the freezing area, i.e. achieving a variable ice ball.

### BRIEF DESCRIPTION OF THE DRAWINGS

Figure 1 is a sectional view of the ablation probe with a threaded split outer sleeve
Figure 2 is a sectional view of the ablation probe with a torsional outer sleeve
Figure 3 is a sectional view of the ablation probe with a Ruhr split outer sleeve
Figure 4 is a sectional view of the ablation probe with an outer sheath regulated outer sleeve
Figure 5 is a schematic view of the surface structure of the outer sheath

Figure: 1- Ablation probe with a threaded split outer sleeve, 101-first inner probe, 102-first outer sleeve, 103-first split member, 104-second split member, 105-first handle, 2-Ablation probe with a torsional outer sleeve, 201-second inner probe, 202-second outer sleeve, 203- third split member, 204-fourth split member, 205-second handle, 3-Ablation probe with a Ruhr split outer sleeve, 301-third inner probe, 302-third outer sleeve, 303-fifth split member, 304-sixth split member, 305-third handle, 4-Ablation probe with an outer sheath regulated outer sleeve, 401-fourth inner probe, 402-fourth outer sleeve, 403-seventh split member, 404-eighth split member, 405-fourth handle, 406-outer sheath, 407-slider, 408-through slot, 409-limit slot, 410- scale identification

### DETAILED DESCRIPTION

The agents referred to in the present invention include hemostatic agents, embolic agents, chemotherapeutic agents, and biological agents, which have positive effects on hemostasis, local chemotherapy, and prevention of implant metastasis. The words "distal end" and "proximal end" refer to the tip of the inner probe and near the end of the handle, respectively. The insulation tube is preferably a vacuum sleeve, aerogel filling, electric film and resistance wire are also optional insulation structure; the insulation tube means electrical insulation, including electric insulation structure by means of electric insulating material or applying electric insulating layer. The inner probe is the cryoablation probe or the probe involving cryoablation and equipped with the outer surface of the inner probe. A preferred scheme is to remove the thermal insulation structure in the cryoablation probe and the ablation probe with cryoablation function, that is, the inner probe is the cryoprobe without the thermal insulation structure of the invention to adjust the size of the ice ball at the distal end of the inner probe. When the inner probe is a cryoelectric ablation probe, the thermal insulation tube should also have an insulating structure; the exposed electrode area or the number of electrodes of the probe can be adjusted by adjusting the outer sleeve, so as to adjust the ablation range. If electrical ablation is required through an thermal insulation tube, the distal end of the outer sleeve is equipped with at least one electrode. The outer sleeve can be used as an electrode for independent electrical ablation, or the outer sleeve and the inner probe can be insulated to form a multi-electrode structure between the inner probe and the outer sleeve for electrical ablation. The electrode is electrically connected with a DC generator and/or an electrical pulse generator. During cryoablation, the outer sleeve can be made to move along the surface of the inner probe axially by splitting the split component, thus isolating and adjusting the ablation range of the inner probe.

The external insulation structure of any one embodiment of the present invention can be a metal material or a non-metallic material, preferably a metal structure, and the metal structure is electroconductive, without direct connection to the electrode, and can be electrically conductive through the metal structure.

The split structure used in the present application can not only be applied to the rigid probe structure, but also to the flexible probe structure, the specific use of flexible probe when the split component should also be flexible, the distal end of the flexible probe may also be provided with balloons, guide wires and other commonly used auxiliary equipment, while the flexible probe can be used with equipment such as laparoscope.

For a better understanding of the invention, the invention is further described below in combination with specific embodiments and figures. However, it will be clear to those skilled in the art that the embodiments of this disclosure can be practiced without these specific details. Further, the particular embodiments of the present disclosure described herein are provided as examples, and should not be used to limit the scope of the present invention to these particular embodiments. In other cases, no well- known materials, components, processes, controller components, software, circuits, timing charts, and / or anatomy are described or illustrated in detail to avoid unnecessary blurring of embodiments.

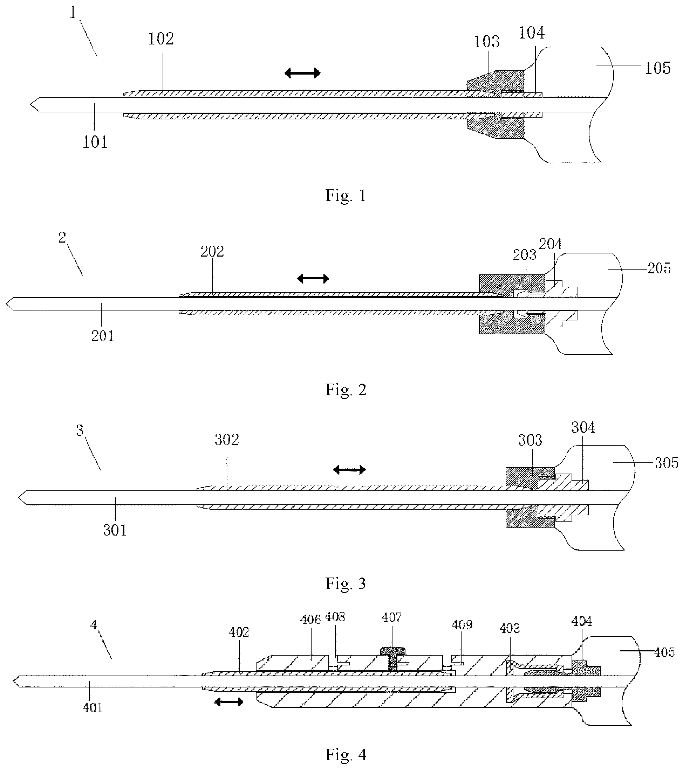

Example 1: Referring to FIG. 1, FIG. 1 is a threaded split outer sleeve ablation probe 1 comprising a first inner probe 101, first outer sleeve 102, the first split member 103, second split member 104, first handle 105 and first inner probe 101 may be a cryoablation probe or cryoelectric ablation probe or cryoelectric ablation probe without a thermal insulation structure, and the surface of the first inner probe 101 has a scale. The first outer sleeve 102 is concentric and coaxially fixed with the first split member 103, the first outer sleeve 102, along with the first split member 103, is bonded to the first inner probe 101 and forms a sliding connection, one end of first handle 105 and one end of second split member 104 are fixed, the first split member 103 and the second split member 104 are threaded connected, the threaded connection structure causes the first split member 103 and the second split member 104 to form a repeatedly split connection structure, when the first split member 103 is connected to the second split member 104, the first outer sleeve 102 and the first inner probe 101 do not slide, note that the threaded connection is only a preferred connection mode, commonly joint structures that can be repeatedly split, such as snap structure, screw structure (connected or separated by rotation) or interference fit structure, can be applied to the invention, for example, refer to FIG. 3, the fifth split member 303 and the sixth split member 304 in FIG. 3 are Ruhr joints, the Ruhr joint can also form a connection structure that can be repeatedly split up. When the first split member 103 and the second split member 104 are split, the first inner probe 101, the second split member 104, and the first handle 105 are sets of devices, and the first outer sleeve 102 and the first split member 103 are sets of devices separated from the previous set of devices. When the first outer sleeve 102 is combined with the cryoablation probe, a thermal insulation tube (preferably a vacuum insulation tube) is selected. If the first outer sleeve 102 is used for electrical ablation, a partially insulated tube with electrodes at the distal end is selected. If the first inner probe 101 is used for electrical ablation, an insulation tube with electrical insulation function is selected as the first outer sleeve 102. If the first inner probe 101 is a cryoelectric ablation probe, the first outer sleeve 102 with thermal insulation and at least partial insulation can be selected. When used, place threaded split outer sleeve ablation probe 1 into the target area, it may then be selected to perform the ablation directly, or to split the first split member 103 and the second split member 104 upon demand, the first inner probe 101 is drawn out so that the first outer sleeve 102 forms a channel between the target area and the outside world, the user may use the first outer sleeve 102 as the guide channel to import drugs to the target area, including but not limited to solid and fluid hemostatic agents, biotherapeutic drugs or chemotherapy drugs, the first inner probe 101 fixed to the first handle 105 is then inserted into the first outer sleeve 102 with the first split member 103, and the first split member 103 and the second split member 104 are combined or mask the area of the first inner probe 101 by deploying the first outer sleeve 102 to regulate the ablation area, and then the ablation of the target area is performed. After ablation, the first split member 103 and the second split member 104 can be separated as required before or during the removal of the threaded split outer sleeve ablation probe 1, and drugs including but not limited to solid and fluid hemostatic agents, biotherapeutic drugs or chemotherapeutic drugs can be injected into the target area through the first outer sleeve 102. Drugs include, but are not limited to, solid and fluid hemostatic agents, biotherapeutic agents, or chemotherapy agents used to stop bleeding, treat, or prevent needle implantation and metastasis of tumors.

Example 2: Referring to FIG. 2, Figure 2 is the torsional outer sleeve ablation probe 2, including a second inner probe 201, a second outer sleeve 202, a third split member 203, a fourth split member 204, and a second handle 205, the second inner probe 201 may be either a cryoablation probe or a cryoelectric ablation probe or a cryoablation probe or a cryoelectric ablation probe provided without an insulation structure, the second outer sleeve 202 is fixed coaxially with the third split member 203, the second outer sleeve 202, along with the third split member 203, is bonded to the second inner probe 201 and forms a sliding connection, one end of the second handle 205 and one end of the fourth split member 204 are fixed, the third split member 203 and the fourth split member 204 adopt non-repeatable connection structures, the third split member 203 and the fourth split member 204 cannot be recoverable, non-repeatable connection structures include, but are not limited to, twisted, fractured, or pulled structures, When the third split member 203 is connected to the fourth split member 204, the second outer sleeve 202 and the second inner probe 201 do not slide. When the third split member 203 and the fourth split member 204 are split, the second inner probe 201, the fourth split member 204 and the second handle 205 are sets of devices, and the second outer sleeve 202 and the third split member 203 are a set of devices detached from the previous set and damaging the third split member prevents the device from connecting again. The second outer sleeve 202 is provided with the cryoablation probe (preferably a vacuum insulation tube), a distal partial insulating tube with an electrode through the second outer sleeve 202, the second outer sleeve 202 by the second inner probe 201, and the second outer sleeve 202 and the second outer sleeve 202 if the second inner probe 201 is at least partially insulated. When used, place torsional outer sleeve ablation probe 2 into the target area, by shielding the area of the second inner probe 201 by deploying the second outer sleeve 202 to regulate the ablation area or directly ablation of the target area, after ablation, the third split member 203 and the fourth split member 204 can be separated as required before or during the removal of the torsional outer sleeve ablation probe 2, and drugs including but not limited to solid and fluid hemostatic agents, biotherapeutic drugs or chemotherapeutic drugs can be injected into the target area through the second outer sleeve 202. Drugs include, but are not limited to, solid and fluid hemostatic agents, biotherapeutic agents, or chemotherapy agents used to stop bleeding, treat, or prevent needle implantation and metastasis of tumors.

Example 3: Referring to FIG. 4, FIG. 4 is an outer sheath regulated outer sleeve ablation probe 4 comprising a fourth inner probe 401, fourth outer sleeve 402, seventh split member 403, eighth split member 404, fourth handle 405, outer sheath 406, slider 407, groove 408, limit slot 409, and scale mark 410, the fourth inner probe 401 may be either a cryoablation probe or a cryoelectric ablation probe or a cryoablation probe or a cryoelectric ablation probe provided without an insulation structure, the fourth outer sleeve 402 is fixed coaxially with the seventh split member 403, the fourth outer sleeve 402, along with the seventh split member 403, is bonded to the fourth inner probe 401 and forms a sliding connection, one end of the fourth handle 405 and one end of the eighth split member 404 are fixed, the seventh split member 403 and the eighth split member 404 can be connected through the repeated split connection structure, commonly joint structures that can be repeatedly split, such as snap structure, screw structure (connected or separated by rotation) or interference fit structure, can be applied to the invention, or optionally connected by non-repeatable connection structure, non-repeatable connection structures include, but are not limited to, twisted, fractured, or pulled structures, when the seventh split member 403 is connected to the eighth split member 404, the fourth outer sleeve 402 and the fourth inner probe 401 do not slide. When the seventh split member 403 and the eighth split member 404 are split, the fourth inner probe 401, the eighth split member 404 and the fourth handle 405 are sets of devices, the fourth outer sleeve 402, the seventh split member 403, and the outer sheath 406 and slider 407 and limit slot 409 are a set of devices detached from the previous set. The outer sheath 406 is firmly connected to the seventh split member 403 and is not fixed over the fourth outer sleeve 402, the outer sheath 406 is provided with a groove 408, the groove 408 can secure the slider 407 through the outer sheath 406 with the fourth outer sleeve 402, Push the slider 407 sliding together with the fourth outer sleeve 402 in the through groove 408, When the slider 407 is rotated the axis of the fourth outer sleeve 402 against the fourth outer sleeve 402, the limit slot 409 may jam the left and right and pull the slider 407 when the fourth outer sleeve 402 is adjusted to the desired position, the limit mode is not limited to pulling the slider, other common ways of limiting positions are also options. Scale marks are provided on the outer sheath 406, 410 mark the relative sliding distance of the fourth outer sleeve 402. The fourth outer sleeve 402 is provided with the cryoablation probe (preferably a vacuum insulation tube), a distal partial insulating tube with an electrode through the fourth outer sleeve 402, the fourth outer sleeve 402 by the fourth inner probe 401, and the fourth outer sleeve 402 and the fourth outer sleeve 402 if the fourth inner probe 401 is at least partially insulated. When used, place outer sheath regulated outer sleeve ablation probe 4 into the target area, it may then be selected to perform ablation directly, or to split the seventh split member 403 and eighth split member 404 on demand, the fourth inner probe 401 is extracted so that the fourth outer sleeve 402 forms a channel between the target area and the outside world, the user can use the fourth outer sleeve 102 as the guiding channel to input drugs to the target area, including but not limited to solid and fluid hemostatic agents and chemicals physical or chemotherapy drugs, then inserting the fourth inner probe 401 fixed to the fourth handle 405 into the fourth outer sleeve 402 with the fourth split member 403 and combining the fourth split member 403 and the eighth split member 404, and the target area is ablated, after ablation, the seventh split member 403 and the eighth split member 404 can be separated as required before or during the removal of the outer sheath regulated outer sleeve ablation probe 4, and drugs including but not limited to solid and fluid hemostatic agents, biotherapeutic drugs or chemotherapeutic drugs can be injected into the target area through the fourth outer sleeve 402. Drugs include, but are not limited to, solid and fluid hemostatic agents, biotherapeutic agents, or chemotherapy agents used to stop bleeding, treat, or prevent needle implantation and metastasis of tumors.

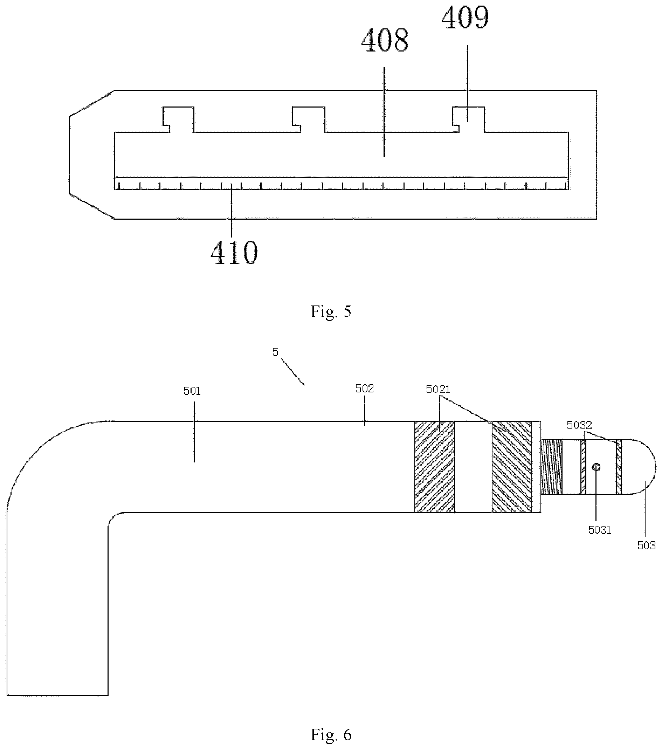

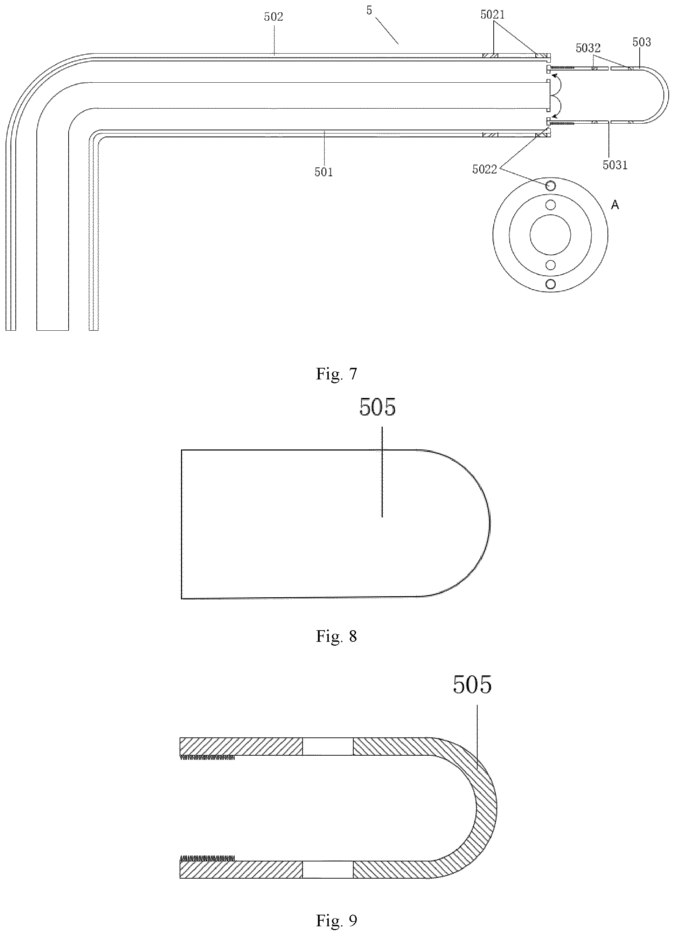

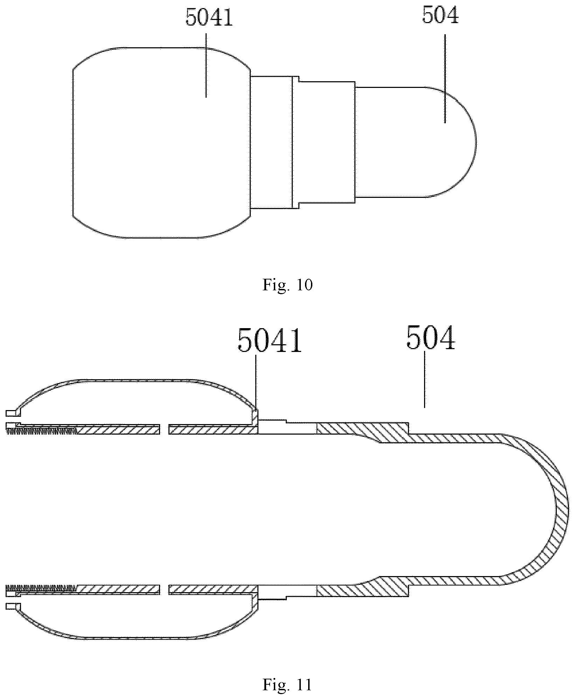

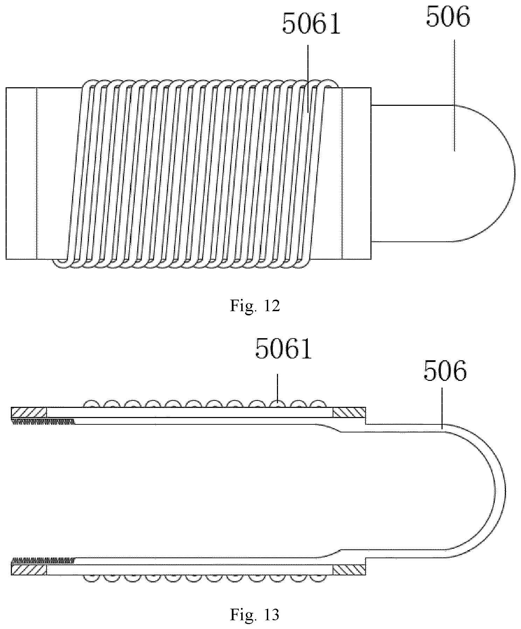

Example 4: Referring to FIG. 6 and 7, FIG. 6 and 7 are flexible ablation probe 5 with a split outer sleeve. The probe including a flexible inner probe 501 and a flexible outer sleeve 502 which both having a flexible structure, the flexible outer sleeve 502 is socketed on the outer surface of the flexible inner probe 501, the specific flexible outer sleeve 502 may be fixed on the surface of the flexible inner probe 501, or may slide along the surface of the flexible inner probe 501; the distal end of the flexible inner probe 501 is a semi-closed structure having a return gas hole 5022, connected to the distal end of the flexible inner probe 501 is the ninth split member 503, as shown in the fitting relationship of FIG. 5, the flexible inner probe 501 and the ninth split member 503 can be regarded as a whole, the fluid tube provided inside the flexible inner probe 501 will be directly connected to the hole structure inside the ninth split member 503 so that the internal fluid can pass into the ninth split member 503, at least one first gas hole 5031 is provided on the ninth split member 503, and the first gas hole 5031 is used to outlet the fluid in the ninth split member 503; the distal end of the flexible inner probe 501 is further provided with a second gas hole 5022 for fluid reflow; the ninth split member 503 is provided with at least one conductive portion 5032, the remaining part should be non-conductive, as shown in the figure of the electrode, the two conductive part 5032 of the ninth split member, can respectively pass the opposite polarity of the current so that the ninth split member 503 can form an electric field, the use of this structure can make the part with the ninth split member 503 have the same effect; in order to avoid direct contact with the target area of the fluid discharged by the ninth split member 503, a closed split member should be selected to connect to the ninth component 503, preferably between the two is provided with a commonly used airtight protective member such as a sealing ring; specifically, the present embodiment selects three compatible split members, the connection method between the two split members may be any disclosed structure, the present embodiment is displayed in a threaded way does not mean only protecting the way of connecting by thread; referring to FIGS. 8 and 9, FIGS. 8 and 9 are sealed split member 505 with electrodes, the main function of the removable fit of the sealed split member 505 and the ninth split member 503 is to ensure that the fluid does not leak, and at the same time achieve the function of combining with cryoablation and / or electrolysis ablation according to the selection of its own material. As shown in FIGS. 8 and 9, the overall structure is selected to conduct heat and at least part of the electric conductive structure, preferably an insulating portion is provided in the middle, and an electric conductive structure is provided on both sides, when the sealed split member 505 with electrodes is fixed in the ninth split member 503, at least one conductive portion 5032 on the ninth split member 503 may generate electrolysis and / or electrical impulses for ablation. Referring to FIG. 10 and 11, FIG. 10 and 11 are split member 504 with a balloon, the balloon 5041 is for supporting, mainly used in blood vessels and urethra and other parts that need to support during the ablation, balloon 5041 is provided with at least two gas hole, corresponding to the first gas hole 5031 and the second gas hole 5022, respectively, the fluid enters the balloon 5041 through the first gas hole 5031 and returns through the second gas hole 5022 into the flexible inner probe 501. The split member 504 with a balloon may also have an electrolysis ablation function, which can be referred to the method in FIG. 6, which is characterized by the fact that the balloon can be used as a conductive portion, and a plurality of conductive portions may be provided on the balloon 5041 and separated by an insulating part to form an electric field on the balloon 5041; referring to FIGS. 12 and 13, FIGS. 12 and 13 are split member 506 with a winding conductor, the outer surface of the split member 506 with a winding conductor 5061 is wound, the conductor 5061 may be used as a thermal ablation and / or electrolysis ablation generator, while the conductor 5061 may include a thermocouple.

The embodiments of the invention are described in detail above, but the contents are only better embodiments of the invention, and cannot be considered to define the scope of implementation of the invention. All equal changes and improvements made in accordance with the scope of the invention shall remain within the scope of the patent.

## Claims

1. An ablation probe having a separable outer sleeve and freezing function, wherein the ablation probe includes an inner probe, an outer sleeve and a handle, the inner probe is slipped connected to the outer sleeve, and one end of the inner probe is fixed with one end of the handle, the ablation probe further comprises a split assembly, the split assembly comprising a first split member and a second split member, the first split member and the second split member are detachable, the first split member is fixed with the outer sleeve, the second split member is partially fixed with the handle, and the outer sleeve is an insulation tube, the inner probe is either a cryoablation probe or a cryoelectric ablation probe with no thermal insulation structure.

2. The ablation probe having a separable outer sleeve of claim 1, wherein the inner probe is either a cryoablation probe or a cryoelectric ablation probe.

3. The ablation probe having a separable outer sleeve of claim 1 or 2, wherein the thermal insulation tube is provided with an electrical insulating layer.

4. The ablation probe having a separable outer sleeve of claim 1 or 2, wherein the distal end of the outer sleeve is provided with at least one electrode, with portions outside of the electrode with an insulating structure.

5. The ablation probe having a separable outer sleeve of claim 1, wherein the surface of the inner probe is provided with marked scale.

6. The ablation probe having a separable outer sleeve of claim 1, wherein the ablation probe further comprises an outer sheath, one end of the outer sheath fixed to the first split member, the outer sheath further comprising a sliding knob fixed through the groove to the outer sleeve.

7. The ablation probe having a separable outer sleeve of claim 6, wherein the outer sheath is provided with a scale and a limit slot.

8. The ablation probe having a separable outer sleeve of claim 1, wherein the detachable structure of the first split member and the second split member is a reusable structure.

9. The ablation probe having a separable outer sleeve of claim 8, wherein the reusable structure is a stuck structure, a spin structure, or a surplus mating structure.

10. The ablation probe having a separable outer sleeve of claim 1, wherein the detachable connection structure of the first split member and the second split member is a non-reusable structure.

11. The ablation probe having a separable outer sleeve of claim 10, wherein the non-reusable structure is a torsional, crushing, or pull-off structure.

12. The ablation probe having a separable outer sleeve of claim 1, wherein: the inner probe, the first and the second split members are flexible structures, and the handle and the inner probe are provided in one.

13. The ablation probe having a separable outer sleeve of claim 12, wherein: the first split member is a balloon connector, the distal end of the inner probe is provided with at least one first gas hole and a second gas hole, the first gas hole is used to input the fluid inside the inner probe into the balloon connector, and the second gas hole is used to return the fluid inside the balloon connection into the inner probe.

14. The ablation probe having a separable outer sleeve of claim 13, wherein: the balloon is at least partially conductive or the surface is provided with a conductive portion.

15. The ablation probe having a separable outer sleeve of claim 13, wherein: the first split member is a thermocouple connector, the thermocouple connector can produce a thermal effect, and the thermal effect can be used for thawing or thermal ablation.

16. The ablation probe having a separable outer sleeve of claim 15, wherein: the thermocouple connector is used for temperature measurement.

17. The ablation probe having a separable outer sleeve of any claims 12 to 16, wherein: the surface of the second split member is provided with at least one electrode.

18. The ablation probe having a separable outer sleeve of any claims 12 to 16, wherein: the surface of the first split member is provided with at least one electrode.

19. An ablation method of an ablation probe having a separable outer sleeve and freezing function of claim 1, by placing the ablation probe into the target area, separating the first and second split members and extracting the inner probe to form the channel with the target area, injecting the agent into or through the sleeve, connecting the first split member or the second split member to adjust the ablation area, and starting the ablation to ablate the target area.

20. The ablation method of claim 19, wherein the first and the second split members are separated and the inner probe is removed and the agent is re-injected into the sleeve.
